# EUROPÄISCHE PATENTSCHRIFT

(11) **EP 4 164 543 B1**
(45) Veröffentlichungstag und Bekanntmachung des Hinweises auf die Patenterteilung: **09.04.2025**
(21) Anmeldenummer: 21746632.5
(22) Anmeldetag: 03.06.2021
(51) Int. Cl.: A61C 8/00, B25B 13/48, B25B 15/00

(54) **TORDIERINSTRUMENT**
TWISTING INSTRUMENT
INSTRUMENT DE TORSION

(30) Priorität: 11.06.2020 DE 102020003492
(43) Veröffentlichungstag der Anmeldung: 19.04.2023
(73) Patentinhaber: Spindler, Bruno, 77866 Oppenau (DE)
(72) Erfinder: REUTER, Timo, 76646 Bruchsal (DE); SPINDLER, Bruno, 77866 Oppenau (DE)
(74) Vertreter: Thämer, Wolfgang
(86) Internationale Anmeldenummer: PCT/DE2021/000105
(87) Internationale Veröffentlichungsnummer: WO 2021/249587

(56) Entgegenhaltungen:
- DE-U1- 9 303 828
- ES-A1- 2 677 998
- US-A1- 2015 289 953

## Beschreibung

Die Erfindung betrifft ein Tordierinstrument für ein Einschraubbauteil mit einer Werkzeugausnehmung, das einen Antriebsschaft, einen Hals und ein Arbeitsteil umfasst.

In der zahnärztlichen Implantologie wird u.a. im Rahmen der Herstellung eines prothetischen Einzelzahnersatzes häufig ein enossaler Implantatkörper verwendet, der die Prothese trägt. In diesem Fall wird der Implantatkörper, eine Art Schraubdübel, in eine künstlich im Patientenkiefer erzeugte Bohrung eingeschraubt. Der eingeschraubte Implantatkörper nimmt bei der fertigen Prothese einen Implantatpfosten auf. Letzterer wird verdrehsicher im Implantatkörper mit einem speziellen Spannmittel, z.B. einer Schraube oder einem speziellen Gewindebolzen, befestigt. Zur Befestigung oder zum Entfernen des Spannmittels wird ein Tordierinstrument verwendet.

Auf den so befestigten Implantatpfosten wird direkt oder indirekt eine, die sichtbare Zahnkrone bildende Suprastruktur, z.B. durch Verkleben, aufgesetzt.

Aus der WO 2017/070 335 A1 ist ein Schraubwerkzeug bekannt, mit dem Schrauben zum Befestigen von Abutments an einem Implantatkörper eingeschraubt werden. Das Schraubwerkzeug hat einen Antriebsteil, eine Welle und einen Arbeitsteil. Das Arbeitsteil hat eine Vielzahl von abgerundeten Zähnen zwischen denen sich konkave Oberflächen befinden.

Die DE 93 03 828 3 U1 offenbart einen Schraubendreher, dessen Arbeitskopf kugelförmig ausgebildet ist und im Querschnitt ein Sechskantprofil aufweist. Der Arbeitskopf geht mit einer Querschnittsverjüngung in den Dreherschaft über. Um diesen gefährdeten Querschnitt nicht allzusehr zu belasten, ist beispielsweise bei einer Ausführung eine Sollbruchstelle zwischen Dreherschaft und Drehergriff vorgesehen. In einer alternativen Ausführung ist eine Torsionsbruchstelle an der Verbindung zwischen Dreherschaft und Drehergriff vorgesehen.

Auch die ES 2 677 998 A1 offenbart einen Schraubendreher mit einem Kugelkopf. Beabstandet zum Kugelkopf weist der Schraubendreher eine schmelzbare Zone auf.

Die US 2015/0289953 A1 zeigt einen Schraubendreher mit einem ellipsoidförmigen, auf den Hals aufgesetzten Arbeitskopf. Die Ausnehmungen der Zahnlücken sind weit in den Hals hineingezogen. Dieser hat zwei kegelstumpfförmige und einen zylindrischen Abschnitt.

Der vorliegenden Erfindung liegt die Problemstellung zugrunde, ein Tordierinstrument für ein Einschraubbauteil so zu verbessern, dass das Arbeitsteil des Tordierinstruments überlastsicher und zuverlässig in die Werkzeugausnehmung des Einschraubteils eingreift, wobei die Mittellinien des Tordierinstruments und des Einschraubbauteils einen Beugewinkel von null oder mehr Winkelgraden einschließen.

Diese Problemstellung wird mit den Merkmalen des Patentanspruchs 1 gelöst. Dabei hat das Arbeitsteil - als ein sechs Zähne aufweisender Kugelkopf - eine kugelförmige Hüllfläche, die mindestens sowohl vom freien Ende als auch von Teilbereichen der zähneeigenen Zahnköpfe des Arbeitsteils kontaktiert wird. Zwischen den Zähnen liegen sechs Zahnlücken. Der Hals oder der Antriebsschaft weist eine Sollbruchzone auf. Der Hals besteht aus einer Halsübergangszone, einer Halshauptzylinderzone, einer Halskonuszone und einer Halsnebenzylinderzone. Außerdem hat der Kugelkopf am Übergang des Kugelkopfs zur Halsnebenzylinderzone einen Hintergriff hat, dessen Radiuskontur tangential in die Halsnebenzylinderzone übergeht.

Mit der Erfindung wird ein Tordierinstrument geschaffen, mit dem bei der Montage und Wartung eines prothetischen Zahnersatzes Einzelteile des Zahnersatzes untereinander verschraubt werden können. Die für die Verschraubung erforderlichen Schrauben, Gewindebolzen oder dergleichen haben eine Werkzeugausnehmung, die mit dem Arbeitsteil eine mechanische Schnittstelle in Form einer winkelbeweglichen Kupplung bildet. Das kugelkopfförmige, verzahnte Arbeitsteil, das z.B. drei bis acht Zähne aufweist, ist über einen zumindest drehelastischen Hals mit einem drehsteifen Antriebsschaft einteilig verbunden. Zur Benutzung des Tordierinstruments wird auf den Antriebsschaft ein i.d.R. manuelles Betätigungselement aufgesteckt. In der zwischen dem Antriebsschaft und dem Betätigungselement gelegenen mechanischen Schnittstelle wird neben Druck und Zug auch ein Drehmoment sowohl für eine Einschraub- als auch für eine Ausschraubbewegung übertragen.

Im primären Ausführungsbeispiel wird direkt in das Tordierinstrument eine zweistufige Drehmomentüberwachung integriert. Für die erste Stufe wird der Hals bezüglich seiner geometrischen Gestalt in Verbindung mit einer entsprechenden Werkstoffauswahl schlank und elastisch ausgebildet. So nimmt der Nutzer in der Endphase der Verschraubungsbewegung über das Betätigungselement eine Torsion von mehr als 10 Winkelgraden wahr, die den Hals zwar elastisch verdrillt, aber keine weitere Einschraubbewegung der Schraube oder des Bolzens bewirkt. Für diese Funktion ist der Hals als Torsionsstab ausgebildet. Er kann dazu ein glatter zylindrischer oder auch kegelstumpfförmiger Stab sein. Ggf. kann der Hals auch zumindest partiell ein Rotationskörper sein, dessen Kontur zumindest abschnittweise auch bogenförmig gestaltet ist. Alle Übergänge einzelner Halsbereiche sind zur Minimierung eventuell auftretender Kerbspannungen ausgerundet.

Die zweite Stufe der Drehmomentüberwachung bildet eine Sollbruchzone. Sie stellt die schwächste Stelle zwischen dem Arbeitsteil und dem Antriebsschaft dar. Bei einem Überschreiten des für die Verschraubung vorgesehenen Drehmoments trennt sich in der Sollbruchstelle der Hals vom Antriebsschaft. Die Bruchstelle liegt hierbei weit außerhalb des schon montierten prothetischen Zahnersatzes, so dass der Hals, zusammen mit dem Arbeitsteil, mit Hilfe einer Pinzette - ohne Kraftauswand - z.B. aus dem Implantatpfosten herausgezogen werden kann.

Weitere Einzelheiten der Erfindung ergeben sich aus den Unteransprüchen und der nachfolgenden Beschreibung schematisch dargestellter Ausführungsformen.
- Figur 1:: Perspektivische Ansicht eines Tordierinstruments;
- Figur 2:: vergrößerte Ansicht des Arbeitsteils nach Figur 1;
- Figur 3:: Längsschnitt des Arbeitsteilhalbzeugs nach der Drehbearbeitung, 20-fach vergrößert;
- Figur 4:: Längsschnitt des Arbeitsteils nach der Nuterzeugung zur Fertigung der Zahnlücken, 20-fach vergrößert;
- Figur 5:: Querschnitt des Arbeitsteils an der Stelle des größten Durchmessers, 20-fach vergrößert;
- Figur 6:: Teilansicht des Instrumentenhalses mit Teilschnitt, 30-fach vergrößert;
- Figur 7:: Seitenansicht eines Formwerkzeugs, 20-fach vergrößert;
- Figur 8:: Eingriff des Arbeitsteils in das Einschraubbauteil im Schnitt durch die Außensechsrundzähne, 20-fach vergrößert;
- Figur 9:: Eingriff des Arbeitsteils in das Einschraubbauteil im Schnitt durch die Außensechsrundzahnlücken, 20-fach vergrößert;
- Figur 10:: wie Figur 8, jedoch schließt die Mittellinie des Arbeitsteils mit der Mittellinie des Einschraubteils einen Beugewinkel von 22 Winkelgraden ein;
- Figur 11:: wie Figur 9, jedoch schließt die Mittellinie des Arbeitsteils mit der Mittellinie des Einschraubteils einen Beugewinkel von 22 Winkelgraden ein;
- Figur 12:: perspektivische Ansicht eines Tordierinstruments mit einer Sollbruchzone in Form einer Ringnut im Antriebsschaft;
- Figur 13:: Längsschnitt zu Figur 12
- Figur 14:: perspektivische Ansicht eines Tordierinstruments mit einer Sollbruchzone in Form einer Rille;
- Figur 15:: Längsschnitt zu Figur 14;
- Figur 16:: perspektivische Ansicht eines Tordierinstruments mit einer Sollbruchzone in Form einer Kerbe;
- Figur 17:: Längsschnitt zu Figur 16;
- Figur 18:: perspektivische Ansicht eines Tordierinstruments mit einer Sollbruchzone in Form eines Konus;
- Figur 19:: Längsschnitt zu Figur 18;
- Figur 20:: perspektivische Ansicht eines Tordierinstruments mit einer Sollbruchzone in Form einer Umlaufnut mit einem kreisabschnittförmigen Querschnitt;
- Figur 21:: Längsschnitt zu Figur 20;
- Figur 22:: perspektivische Ansicht eines Tordierinstruments mit einer Sollbruchzone in Form einer Mattheck-Doppel-Kontur;
- Figur 23:: Längsschnitt zu Figur 22;
- Figur 24:: perspektivische Ansicht wie Figur 22, jedoch mit einer die Mattheck-Doppel-Kontur umgebende Schutzhülse;
- Figur 25:: Längsschnitt zu Figur 24;
- Figur 26:: perspektivische Ansicht wie Figur 20, jedoch mit Handgriff;
- Figur 27:: Längsschnitt zu Figur 26.

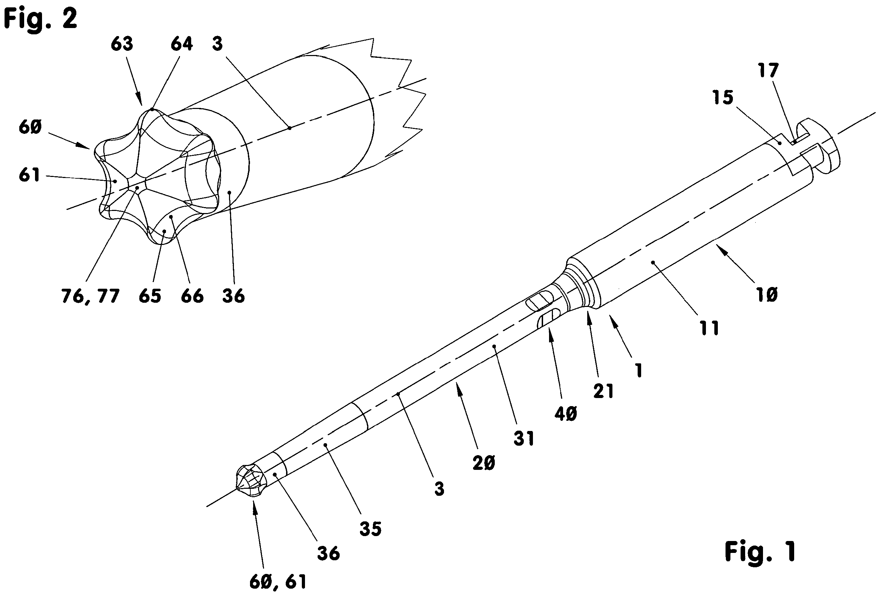

Die Figur 1 zeigt ein zahntechnisches Tordierinstrument (1), das aus einem zumindest bereichsweise genormten Antriebsschaft (10), einem schlanken Instrumentenhals (20) und einem Außensechsrundkugelkopf (61) als Arbeitsteil (60) besteht. Mit dem Tordierinstrument (1) lässt sich im Rahmen einer zahnärztlichen Implantologie zur Herstellung eines prothetischen Zahnersatzes eine Prothese mit einem Implantatkörper verschrauben.

Das einteilige Tordierinstrument (1) ist z.B. aus einer austenitischen Legierung auf Kobaltbasis mit der Werkstoffbezeichnung COCr20Ni15Mo7 hergestellt. Dieser biokompatible unmagnetische Werkstoff hat eine hohe Streckgrenze und ist beständig gegen Korrosion und Wasserstoffversprödung. Er ist alterungsbeständig und besitzt eine große Ermüdungsfestigkeit.

Das Tordierinstrument (1) wird bei 530°C für z.B. drei Stunden in Argon oder Hochvakuum gehärtet und im Nachgang poliert. Je nach Länge des Härteprozesses liegt der werkstoffbedingte Schubmodul zwischen 75 und 82 GPa.

Ein alternativer Werkstoff ist der Schnellarbeitsstahl HS2-9-1-8. Seine wesentlichen Legierungsbestandteile sind im Mittel neben 1 % Kohlenstoff, 2 % Wolfram, 9 % Molybdän, 1 % Vanadium und 8 % Kobalt.

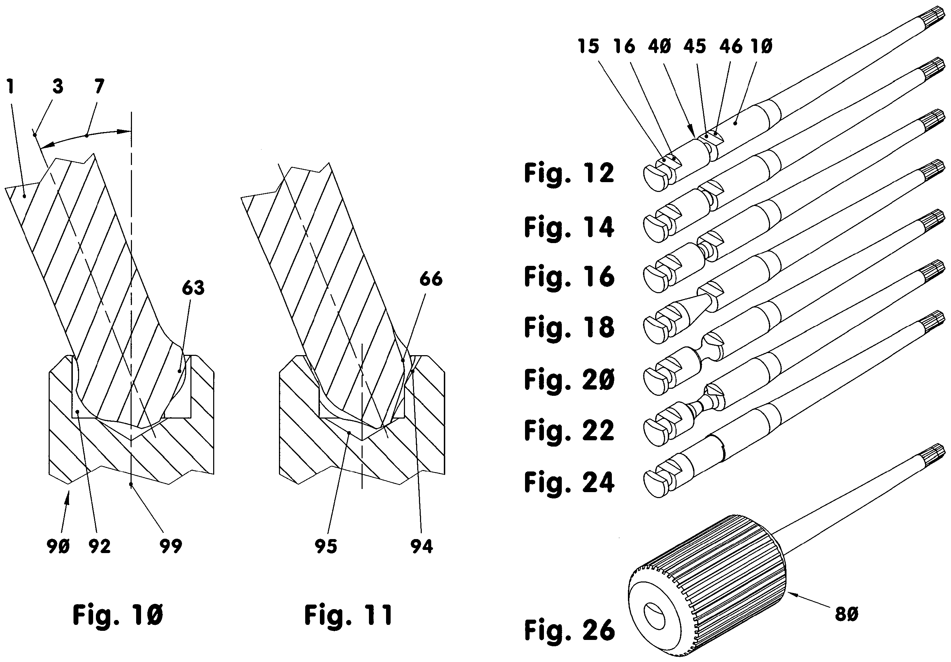

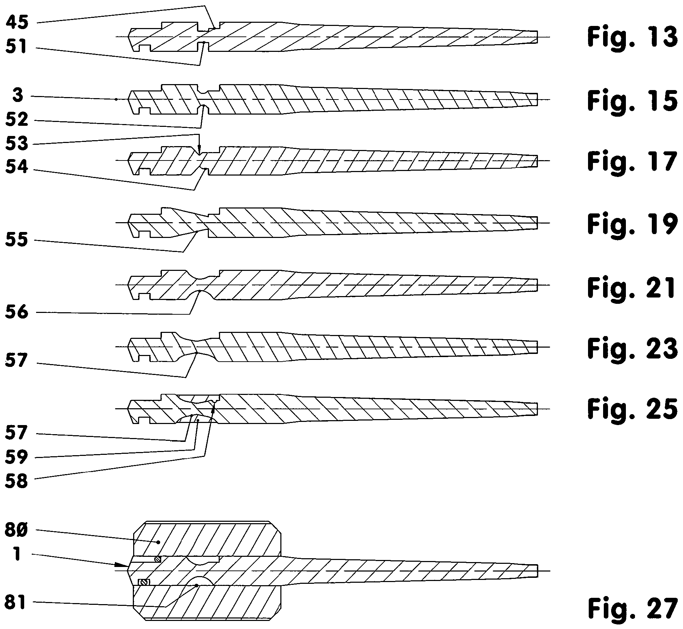

Der Antriebsschaft (10) des Tordierinstruments (1) ist ein Werkzeugabschnitt, der für eine rotierende oder oszillierende Bewegung ausgelegt ist. Er besitzt einen geraden zylindrischen Schaftabschnitt (11), der an seinem freien Ende eine Abflachung (41) und eine Rille (17) aufweist. Der Durchmesser des Schaftabschnitts misst 2,35 mm. Er hat im Ausführungsbeispiel eine Länge von 12,5 mm. Auf den Schaftabschnitt (11) wird zur Verwendung des Tordierinstruments (1) ein Handstück (80) in Form eines austauschbaren Griffes gesteckt, vgl. Figuren 26 und 27. Die Abflachung (41) dient dabei der Übertragung des in das Handstück (80) manuell eingeleiteten Drehmoments auf das Tordierinstrument (1). In die als Hintergriff dienende Rille (17) des Schaftabschnitts (11) greift ein im Handstück (80) federnd gelagerter Stift, der das Tordierinstrument (1) verliersicher im Handstück (80) hält. Dieser Schaftabschnitt (11) ist in der DIN EN ISO 1779 als Typ 1 beschrieben.

Selbstverständlich kann im Handstück (80) auch ein Ratschenmechanismus integriert sein. Dieser Mechanismus ermöglicht es, z.B. in einem begrenzten Arbeitsraum, durch wiederholte, sektorweise Schwenkbewegungen des Handstücks (80) eine Drehbewegung in einem bestimmten Drehsinn in das Einschraubbauteil (90) einzuleiten. Zudem kann der Ratschenmechanismus mit einer Drehmomentbegrenzung kombiniert werden.

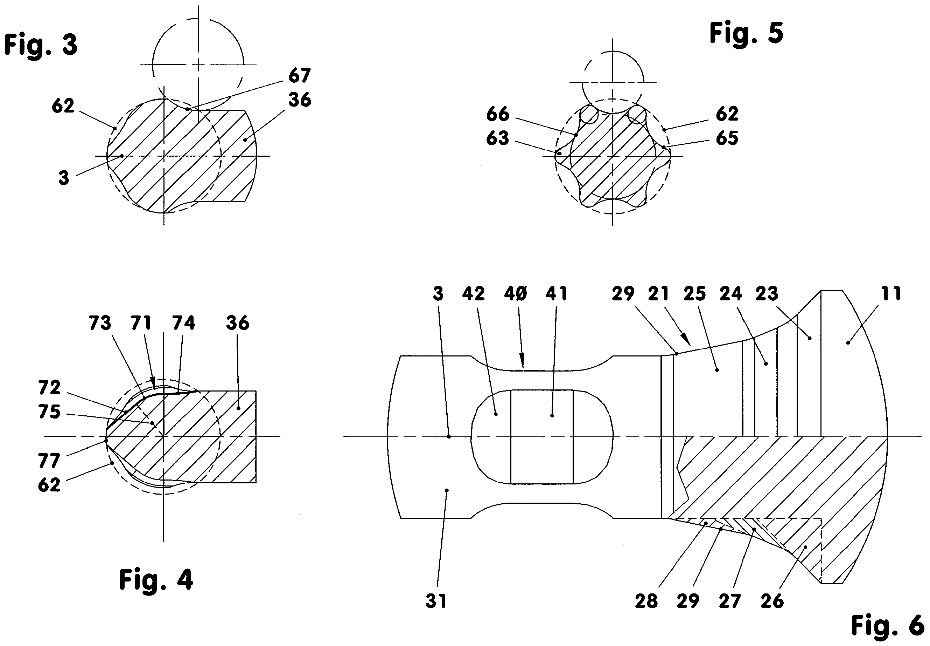

An den Schaftabschnitt (11) schließt sich ein z.B. 16,4 mm langer Instrumentenhals an, der im Weiteren Hals (20) genannt wird. Der Hals (20) besteht aus einer Halsübergangszone (21), einer Halshauptzylinderzone (31), einer Halskonuszone (35) und einer Halsnebenzylinderzone (36). In der Halsübergangszone (21) wird der Schaftabschnitt (11) zur Halshauptzylinderzone (31) hin verjüngt. Es findet eine Durchmesserreduzierung von z.B. 2,35 mm auf z.B. 1,3 mm statt. Um die durch den Durchmessersprung entstehende Kerbspannung so klein wie möglich zu halten, wird die Halsübergangszone (21) mithilfe der Mattheck'schen Zugdreieckmethode gestaltet, vgl. Figur 6.

Die Figur 6 zeigt die zwischen dem Schaftabschnitt (11) und der Sollbruchzone (40) gelegene Halsübergangszone (21). In der oberen, nicht geschnittenen Hälfte der Figur 6 ist zu erkennen, dass die Halsübergangszone (21) aus einem ersten (23), zweiten (24) und dritten Übergangszonenbereich (25) besteht. Dabei hat der erste, sich an den Schaftabschnitt (11) anschließende Übergangszonenbereich (23), einen sich verjüngenden geraden Konus mit einem Kegelwinkel von 90 Winkelgraden. Der zweite, sich an den ersten Übergangszonenbereich (23) anschließende Übergangszonenbereich (24) hat einen sich verjüngenden geraden weiteren Konus mit einem Kegelwinkel von 45 Winkelgraden. Auch der dritte, sich an den zweiten Übergangszonenbereich (24) anschließende Übergangszonenbereich (25) weist einen sich wiederum verjüngenden geraden Konus mit einem Kegelwinkel von 22,5 Winkelgraden auf. Zwischen den einzelnen Koni ist jeweils ein Übergangsradius von z.B. 0,5 mm vorgesehen.

Die Länge der einzelnen Übergangszonenbereiche (23-25) ergibt sich aus der Konstruktion mittels der - in der Schnittfläche nach Figur 6 unterschiedlich schraffierten - drei gestrichelt dargestellten Zugdreiecke (26-28). Alle drei Zugdreiecke bilden jeweils ein gleichschenkliges Dreieck. Das erste Zugdreieck (26), es ist rechtwinklig, hat eine Hypotenuse, die sich wie die Kontur einer 45°-Fase von der Kante des Schaftabschnitts (11) auf das Niveau des Außendurchmessers der Halshauptzylinderzone (31) erstreckt. An der mittelliniennahen Hälfte der Hypotenuse des ersten Zugdreiecks (26) schließt sich das spitzwinkelige zweite Zugdreieck (27) mit seiner dem Schaftabschnitt (11) nächstgelegenen Kathete an. Der zwischen der Hypotenuse und einer Kathete eingeschlossene Winkel misst 22,5 Winkelgrade. Das dritte Zugdreieck (28) schmiegt sich mit seiner dem Schaftabschnitt (11) nächstgelegenen Kathete an der mittelliniennahen Hälfte der Hypotenuse des zweiten Zugdreiecks (27) an. Seine andere Kathete befindet sich auf dem Niveau des Außendurchmessers der Halshauptzylinderzone (31). Die beiden halben Hypotenusen der Zugdreiecke (26) und (27) bilden zusammen mit der Hypotenuse des dritten Zugdreiecks (28) die kerbspannungsarme Mattheck'sche Kontur (29). Selbstverständlich kann die Mattheck'sche Kontur (29) durch eine stetige Kurve angenähert werden.

Die zylindrische Halshauptzylinderzone (31) hat eine Länge von z.B. 10,15 mm.

Im hinteren Bereich der Halshauptzylinderzone (31) befindet sich eine Sollbruchzone (40). Die Mitte der Sollbruchzone (40) liegt z.B. 2,25 mm vor dem Schaftabschnitt (11). Die Sollbruchzone (40) weist z.B. drei bis sechs äquidistant auf dem Umfang der Halshauptzylinderzone (31) verteilt angeordnete Abflachungen (41) auf. Nach den Figuren 1 und 6 werden im Ausführungsbeispiel vier Abflachungen gewählt. Jede Abflachung ist eben ausgebildet und hat die Form eines Rechtecks. Die Rechteckkantenlänge, die parallel zur Mittellinie (3) ausgerichtet ist, hat eine Länge von z.B. 0,5 mm. Die quer zur Mittellinie (3) liegende Rechteckkantenlänge misst z.B. 0,75 mm. Die entlang dem Umfang nebeneinanderliegenden Abflachungen (41) der Sollbruchzone (40) kontaktieren sich gegenseitig nicht, obwohl ihre Flächenschwerpunkte auf einer Ebene liegen, die von der Mittellinie (3) senkrecht geschnitten wird. Die Abflachungen bilden somit ein Vierkantprofil mit abgerundeten Eckbereichen.

An die quer zur Mittellinie (3) liegenden Rechteckkanten schließen sich - in Längsrichtung des Tordierinstruments (1) - jeweils Abflachungsausläufe (42) an, deren konkav gekrümmte Flächen hier Teilflächen eines Zylinders sind, dessen einzelner Durchmesser jeweils z.B. 1 mm misst.

Der Querschnitt der Sollbruchzone (40) ist um mindestens 18 % kleiner als der reguläre Querschnitt der Halshauptzylinderzone (31).

Die vorn an die Halshauptzylinderzone (31) anschließende Halskonuszone (35) reduziert ihren Durchmesser zum Arbeitsteil (60) hin auf z.B. 1,1 mm. Die Halskonuszone (35) schließt ausgehend von der Halshauptzylinderzone (31) einen Konuswinkel von 2,86 Winkelgraden ein. Die Halskonuszone (35) hat dabei eine Länge von z.B. 4 mm. An sie schließt sich eine z.B. 1 mm lange Halsnebenzylinderzone (36) an, die sich bis zum Arbeitsteil (60) hin erstreckt.

Die Figuren 12-27 zeigen Tordierinstrumente, die im Bereich des nach den Figuren 26 und 27 im Handstück (80) steckenden Antriebsschafts (10) verschiedene Sollbruchzonen (40) aufweisen. Vor der jeweiligen Sollbruchzone (40) ist eine zweite, 0,9 mm breite Antriebsabflachung (45) angeordnet, deren Abstand von der Mittellinie (3) dem Mittellinienabstand der ersten Antriebsabflachung (15) entspricht. Die zweite Antriebsabflachung (45) hat die Aufgabe, nach einem Bersten der Sollbruchzone (40) hilfsweise als Antriebsfläche zur Einleitung eines Drehmoments zu dienen. Die zweite Antriebsabflachung (45) endet vorn - wie auch die erste Antriebsabflachung (15) - in einer z.B. planen Stirnanschlagfläche (46). Beide Stirnanschlagflächen (16, 46) sind z.B. 4,8 mm voneinander beabstandet.

In den Figuren 12 und 13 befindet sich unmittelbar hinter der zweiten Antriebsabflachung (45) als Sollbruchzone eine z.B. 0,725 mm tiefe und z.B. 0,9 mm breite Ringnut (51). Die Ringnut (51) hat einen rechteckigen Querschnitt.

Die Figuren 14 und 15 zeigen anstelle der Ringnut (51) eine gleichbreite Rille (52), deren Rillengrundkontur ein Halbkreis ist. Die Rille (52) ist ebenfalls 0,725 mm tief.

Die Sollbruchzone der Figuren 16 und 17 besteht aus einem kurzen Zylinderabschnitt (54) und einer Kerbe (53), deren Kerbflanken mit der Mittellinie (3) jeweils einen Winkel von 45 Winkelgraden einschließen. Der vor der scharfen Kerbe (53) gelegene Zylinderabschnitt (54) hat bei einem Durchmesser von z.B. 1,35 mm eine Länge von 0,53 mm. Der Minimaldurchmesser der Sollbruchzone beträgt im Bereich der 90°-Kerbe 0,9 mm. Dieser Minimaldurchmesser gilt für alle Varianten der Figu-ren 12-27.

Die Figuren 18 und 19 zeigen zwischen der zweiten (45) und der ersten Antriebsabflachung (15) einen z.B. 3,21 mm langen Kerbkonus (55), dessen Konuswinkel z.B. 24,4 Winkelgrade beträgt. Die schmalste Stelle des Kerbkonus (55) endet in einer planen Teilringfläche unmittelbar vor der zweiten Antriebsabflachung (45).

In den Figuren 20 und 21 bildet die Sollbruchzone eine Umlaufnut (56) mit einem kreisabschnittförmigen Querschnitt. Der Radius der Nutkontur misst z.B. 1,25 mm. Zum Antriebsschaft (10) hin gibt es einen Übergangsradius von z.B. 0,25 mm.

Jede Sollbruchzone der Figuren 22-25 ist eine umlaufende Nut mit einer Mattheck-Doppel-Kontur (57). Die einfache Mattheck'sche Kontur (29) ist im Zusammenhang mit der Halsübergangszone (21) der Figur 6 beschrieben. Im Fall der Figu-ren 22-25 werden zwei Mattheck'sche Konturen (29) so nebeneinander angeordnet bzw. gespiegelt, dass sich die schmalsten Zugdreiecke beider Konturen an ihren spitzwinkeligen Ecken kontaktieren.

Die Sollbruchzone der Figuren 24-25 ist mit einer Schutzhülse (59) umgeben, die die umlaufende Nut (57) ausfüllt. Die zylindrische Außenwandung der Schutzhülse (59) hat einen Durchmesser, der z.B. 0,15 mm kleiner ist als der Durchmesser des Antriebsschafts (10). Um die Schutzhülse (59) verliersicher am vorderen Teil des Antriebsschafts (10) zu halten, befindet sich in der zweiten Antriebsabflachung (45) eine kleine Halteausnehmung (58), z.B. in Form einer kurzen Bohrung, in die der Werkstoff der Schutzhülse (59) formschlüssig eingreift.

Bei einem Bruch der Sollbruchzone bleibt auf diese Weise die Schutzhülse (59) am vorderen Teil des Tordierinstruments (1) haften, sodass beim Herausrutschen des Handstücks (80) für den Patienten keine Verletzungsgefahr besteht.

Die Figuren 26 und 27 zeigen ein Handstück (80), in das das in Figur 20 dargestellte Tordierinstrument verlier- und verdrehsicher eingesteckt ist. In der Schnittdarstellung der Figur 27 ist deutlich zu erkennen, dass sich die Sollbruchzone im mittleren Bereich der Aufnahmebohrung (81) des Handstücks (80) befindet. Auf diese Weise ist sichergestellt, dass bei einem Bruch der Sollbruchzone keine Verletzungsgefahr für den Nutzer des Tordierinstruments (1) ausgeht.

Das Arbeitsteil (60) ist ein Außensechsrundkugelkopf (61). Letzterer ist geeignet, in eine Werkzeugausnehmung (92) eines Einschraubbauteils (90) mit Innensechsrund nach DIN EN ISO 10664 drehmomentübertragend einzugreifen. Der Außensechsrundkugelkopf (61) weist am Umfang sechs Zähne (63) auf, zwischen denen sechs Zahnlücken (66) liegen, vgl. auch Figur 2. Die Zahnköpfe (64) der Zähne (63) haben Kopfrundungen, die unmittelbar in die Zahnfußrundungen im Bereich der Zahnlücken (66) übergehen. Das Arbeitsteil (60) überträgt das Drehmoment auf den Innensechsrund (92) des Einschraubbauteils (90) über die Flanken (65) seiner Zähne (63).

Als Arbeitsteilhalbzeug besteht das Arbeitsteil (60) aus einer am vorderen Ende des Halses (20) bzw. der Halsnebenzylinderzone (36) angeformten - auf der Mittellinie (3) gelegenen - Kugel, vgl. Figur 3. Die Oberfläche dieser Kugel stellt beim fertigen Produkt die in den Figuren 3-5 gestrichelt eingezeichnete Hüllfläche (62) dar. Sie hat einen Durchmesser von z.B. 1,38 mm

In einem ersten Arbeitsgang wird, gemäß Figur 3, am Übergang zur Halsnebenzylinderzone (36) ein Hintergriff (67) erzeugt, der es ermöglicht, die Mittellinie (3) des Tordierinstruments (1) um einen Beugewinkel (7) von bis zu 22 Winkelgraden gegenüber der Mittellinie (99) des Einschraubbauteils (90) zu kippen. Der Hintergriff (67) hat eine Radiuskontur, dessen Radius z.B. 0,55 mm misst. Die Radiuskontur geht tangential in die Halsnebenzylinderzone (36) über. Das Zentrum der Radiuskontur sitzt z.B. 0,4 mm hinter dem auf der Mittellinie (3) liegenden Mittelpunkt der Hüllfläche (62).

In einem weiteren Arbeitsgang wird mithilfe des Formwerkzeugs (100) die Kugel des Arbeitsteilhalbzeugs verzahnt, vgl. Figur 4. Mit dem Formwerkzeug (100) wird die Verzahnung, je nach Werkzeugtyp, durch Schleifen oder Fräsen spanabhebend bearbeitet oder in einem Rollvorgang umgeformt.

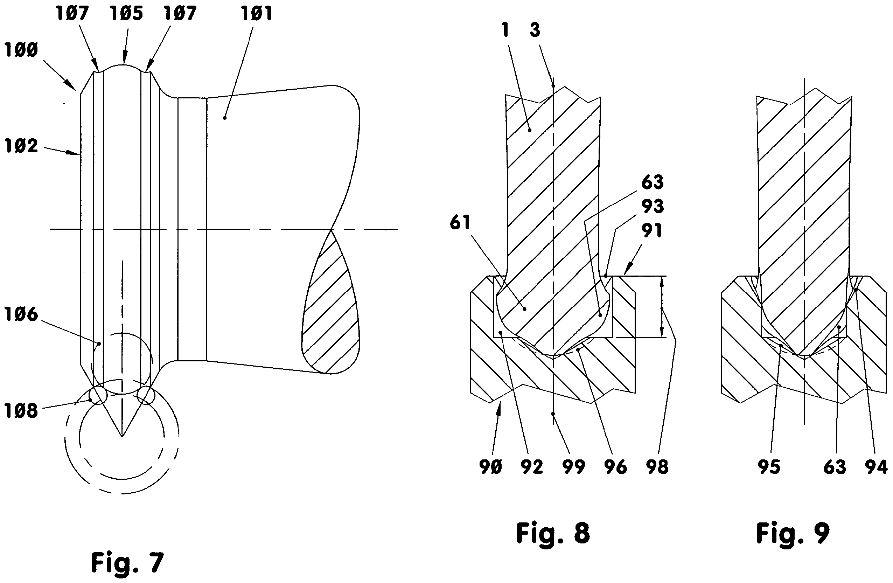

Das Formwerkzeug (100) hat dazu eine an einem Werkzeugschaft (101) angeformte Profilscheibe (102), vgl. Figur 7. Die Profilscheibe (102), die einen Durchmesser von z.B. 4 mm hat, weist ein mittleres Zahnlückenprofil (105) und zwei randseitige Zahnkopfprofile (107) auf. Das Zahnlückenprofil (105) ist Teil einer Torusoberfläche, dessen Durchmesser des Lückenkreises (106) z.B. 0,74 mm beträgt. Die randseitigen Zahnkopfprofile (107) sind jeweils ebenfalls Teil einer Torusoberfläche, deren Durchmesser der Zahnkopfkreise (108) z.B. 0,22 mm misst. Die Mittelpunkte der Zahnkopfkreise (108) liegen zum einen jeweils um z.B. 0,29 mm - parallel zur Mittellinie (3) - beabstandet zum Mittelpunkt des Lückenkreises (106). Zum anderen sind sie zum Mittelpunkt des Lückenkreises (106) um 0,384 mm radial nach außen versetzt angeordnet. Die Zahnkopfkreise (108) kontaktieren jeweils den Lückenkreis (106) in einem Berührpunkt. In den beiden Berührpunkten haben beide Kreise (106, 108) identische Tangenten.

Zum Formen der sechs Zähne (63) und der entsprechenden Zahnlücken (66) wird das Formwerkzeug (100) entlang einer in Figur 4 dargestellten Zahnlückengrundkontur (71) geführt. Letztere besteht aus einem vorderen, geradlinigen Lückengrundbereich (72), einem mittleren, bogenförmigen Lückengrundbereich (73) und einem hinteren, ebenfalls bogenförmigen Lückengrundbereich (74). Alle drei Lückengrundbereiche (72-74) gehen tangential ineinander über.

Der mittlere Lückengrundbereich (73) ist ein Kreisbogen, der einen Winkel von 40 Winkelgraden und 26 Winkelminuten überdeckt.

Der Kreisbogen hat einen Radius von z.B. 0,515 mm. Der Mittelpunkt des mittleren Lückengrundbereichs (73) entspricht dem Mittelpunkt der kugelförmigen Hüllfläche (62). Der vorderste Punkt des mittleren Lückengrundbereiches (73) liegt auf einem fiktiven Radiusstrahl (75), der mit der Mittellinie (3) einen Winkel von 50 Winkelgraden einschließt.

Im vordersten Punkt des mittleren Lückengrundbereiches (73) schließt sich der geradlinige vordere Lückengrundbereich (72) an. Letzterer endet am freien Ende (76) des Arbeitsteils (60). Der geradlinige Lückengrundbereich (72) schließt mit der Mittellinie (3) einen Winkel von 40 Winkelgraden ein.

Am hinteren Ende des mittleren Lückengrundbereiches (73) beginnt der hintere Lückengrundbereich (74). Er hat einen Radius von z.B. 2,175 mm. Der hintere Lückengrundbereich (74) läuft im Hintergriff (67) des Arbeitsteils (60) aus.

Nach jedem Abfahren der Zahnlückengrundkontur (71) wird das Arbeitsteilhalbzeug für den nächsten Formvorgang um 60 Winkelgrade weitergeschwenkt. Auf diese Weise entsteht eine Verzahnung, wie sie in Figur 5 als Querschnitt durch den Mittelpunkt der Hüllfläche (62) und in Figur 2 in einer perspektivischen Ansicht dargestellt ist. Nach Figur 2 ist die Spitze (76) des Arbeitsteils (60) ein Kopfstern (77), dessen kleinster Durchmesser z.B. 0,18 mm misst. Die Wölbung des Kopfsterns (77) entspricht der Wölbung der Hüllfläche (62).

Alle Maße beziehen sich auf einen Innensechsrund für Schrauben nach DIN EN ISO 10664, Typ Nr. 5.

Das Einschraubbauteil (90) ist abhängig vom Abutmenttyp eine Schraube, ein Gewindebolzen oder dergleichen. Der Kopf der Schraube oder mindestens eines der Enden des Gewindebolzens hat eine Werkzeugausnehmung (92), die ein Innensechsrund nach DIN EN ISO 10664 darstellt, vgl. Figuren 8-11. Nach diesen Figuren weist die Werkzeugausnehmung (92) im Bereich ihrer Öffnung (93) eine 30°-Fase (94) auf, deren Breite kleiner ist als die halbe Tiefe (98) des Nutzbereichs der Werkzeugausnehmung (92). Die Breite der 30°-Fase (94) beträgt nach den Figuren 9 und 11 z.B. 0,36 mm.

Die Werkzeugausnehmung (92) hat einen Boden (95), der die Form eines geraden Kegels aufweist, dessen Kegelwinkel z.B. 118 Winkelgrade misst und dessen Spitze (76) weiter von der Öffnung (93) der Werkzeugausnehmung (92) entfernt liegt als die Kegelgrundlinie. Auf diesem Boden (95) stützt sich das Tordierinstrument (1) bei jedem Ein- oder Ausschraubvorgang des Einschraubbauteils (90) ab, um eine sichere Anlage des Arbeitsteils (60) in der Werkzeugausnehmung (92) zu gewährleisten.

Alternativ kann in der Werkzeugausnehmung (92) auch ein Boden (96) vorgesehen werden, dessen Bodenfläche sphärisch gekrümmt ist, vgl. gestrichelt gezeichneter Bogen in den Figuren 9 und 10. Die Krümmung des Bodens (96) hat einen Radius von z.B. 0,7 mm. Der Mittelpunkt der Krümmung liegt z.B. 0,265 mm unterhalb der planen, die Werkzeugausnehmung (92) umgebenden Stirnfläche des Einschraubbauteils (90). Ggf. kann der Boden auch eine anders gekrümmte Rotationsfläche oder sogar eine tiefergesetzte Planfläche sein.

Zum Einschrauben des Einschraubbauteils (90) - in den in den Figuren nicht dargestellten Implantatkörper - wird das Tordierinstrument (1) mit seinem Arbeitsteil (60) in die Werkzeugausnehmung (92) des Einschraubbauteils (90) eingeführt. Dabei greifen zum einem die Zähne (63) des Arbeitsteils (60) in die Zahnlücken der Werkzeugausnehmung (92) mit minimalem Spiel ein. Zum anderen legt sich die Spitze (76) bzw. der Kopfstern (77) des Arbeitsteils (60) am Boden (96) des Einschraubbauteils (90) an, vgl. Figuren 8-11.

In den Figur 8 und 10 ist das Arbeitsteil (60) des Tordierinstruments (1) im Bereich der Zahnköpfe (64) der Außensechsrundzähne (63) geschnitten, während in den Figuren 9 und 11 das Einschraubbauteil (90) um idealerweise z.B. 30 Winkelgrade um seine Mittellinie (99) geschwenkt dargestellt ist. Demnach ist auch das Tordierinstrument (1) ebenfalls idealerweise um z.B. 30 Winkelgrade um seine Mittellinie (3) geschwenkt worden.

Der mit zunehmendem Beugewinkel (7) größer werdende Kardanfehler bewirkt zumindest theoretisch einen Versatz und eine Winkeländerung der Mittellinie (99) des Tordierinstruments (1) zwischen den Figuren 8 und 9 sowie 10 und 11. Dies wird hier vernachlässigt.

Gegen Ende des Einschraubvorgangs nimmt das Anzugsdrehmoment sprunghaft zu. Um ein Erreichen des maximalen Anzugsdrehmoments am Handstück (80) des Tordierinstruments (1) für den Nutzer haptisch erfahrbar zu machen, tordiert sich der Hals (20) des Tordierinstruments (1) nach Figur 1 um 10-12 Winkelgrade, ohne dass sich in der Sollbruchzone (40) eine messbare plastische Verformung ergibt.

### Bezugszeichenliste:

- 1: Tordierinstrument, Schraubendrehinstrument
- 3: Mittellinie
- 7: Beugewinkel, Winkel zwischen (3) und (99)

- 10: Antriebsschaft
- 11: Schaftabschnitt, zylindrisch
- 15: Antriebsabflachung, erste
- 16: Stirnanschlagfläche
- 17: Rille, Nut

- 20: Hals, Instrumentenhals
- 21: Halsübergangszone
- 23: erster Übergangszonenbereich
- 24: zweiter Übergangszonenbereich
- 25: dritter Übergangszonenbereich
- 26: erstes Zugdreieck, rechtwinkelig
- 27: zweites Zugdreieck
- 28: drittes Zugdreieck
- 29: Mattheck'sche Kontur

- 31: Halszylinderzone, Halshauptzylinderzone

- 35: Halskonuszone, Halsabschnitt
- 36: Halszylinderzone, Halsnebenzylinderzone, Halsabschnitt

- 40: Sollbruchzone
- 41: Abflachungen, vier, plan
- 42: Abflachungsausläufe
- 45: Antriebsabflachung, zweite
- 46: Stirnanschlagfläche

- 51: Ringnut
- 52: Rille
- 53: Kerbe, 90°-Kerbe
- 54: Zylinderabschnitt
- 55: Kerbkonus, Konus
- 56: Umlaufnut
- 57: Mattheck-Doppel-Kontur; Nut, umlaufend
- 58: Halteausnehmung
- 59: Schutzhülse

- 60: Arbeitsteil
- 61: Kugelkopf, Außensechsrundkugelkopf
- 62: Hüllfläche, kugelförmig, Kugel
- 63: Zähne, Außensechsrundzähne
- 64: Zahnköpfe
- 65: Flanken, Zahnflanken
- 66: Zahnlücken
- 67: Hintergriff

- 71: Zahnlückengrundkontur
- 72: vorderer Lückengrundbereich, geradlinig
- 73: mittlerer Lückengrundbereich, bogenförmig
- 74: hinterer Lückengrundbereich, bogenförmig
- 75: Radiusstrahl von (60), fiktiv
- 76: freies Ende, Spitze
- 77: Kopfstern

- 80: Handstück, Griff, Betätigungselement
- 81: Aufnahmebohrung

- 90: Einschraubbauteil, Schraube
- 91: Kopf, Innensechsrundkopf
- 92: Werkzeugausnehmung, Innensechsrund, Torx ^{®}
- 93: Öffnung
- 94: Fase, 30°-Fase
- 95: Boden von (92), kegelmantelförmig
- 96: Boden, sphärisch gekrümmt
- 98: Tiefe, Eindringtiefe von (92)
- 99: Mittellinie von (90)

- 100: Formwerkzeug
- 101: Werkzeugschaft
- 102: Profilscheibe
- 105: Zahnlückenprofil, Mitte
- 106: Lückenkreis, Kreis
- 107: Zahnkopfprofil, randseitig
- 108: Zahnkopfkreise, Kreise

## Patentansprüche

1. Tordierinstrument für ein Einschraubbauteil (90) mit einer Werkzeugausnehmung (92), das einen Antriebsschaft (10), einen Hals (20) und ein Arbeitsteil (60) umfasst,
- wobei das Arbeitsteil (60) - als ein sechs Zähne (63) aufweisender Kugelkopf (61) - eine kugelförmige Hüllfläche (62) hat, die mindestens sowohl vom freien Ende (76) als auch von Teilbereichen der zähneeigenen Zahnköpfe (64) des Arbeitsteils (60) kontaktiert wird,
- wobei zwischen den Zähnen (63) sechs Zahnlücken (66) liegen,
- wobei der Hals (20) oder der Antriebsschaft (10) eine Sollbruchzone (40) aufweist,
- wobei der Hals (20) aus einer Halsübergangszone (21), einer Halshauptzylinderzone (31), einer Halskonuszone (35) und einer Halsnebenzylinderzone (36) besteht und
- wobei der Kugelkopf (61) am Übergang des Kugelkopfs (61) zur Halsnebenzylinderzone (36) einen Hintergriff (67) hat, dessen Radiuskontur tangential in die Halsnebenzylinderzone (36) übergeht.

2. Tordierinstrument gemäß Anspruch 1, **dadurch gekennzeichnet, dass** der Antriebsschaft (10) nach DIN EN ISO 1797 zum Typ 1 zählt, der an seinem freien Ende mit einer Antriebsabflachung (15) und einer Rille (17) ausgestattet ist.

3. Tordierinstrument gemäß Anspruch 1, **dadurch gekennzeichnet, dass** die Halsübergangszone (21) einen ersten (23), zweiten (24) und dritten Übergangszonenbereich (25) umfasst, wobei der erste sich an den Antriebsschaft (10) anschließende Übergangszonenbereich (23), ein sich verjüngender gerader Konus mit einem Kegelwinkel von 90 Winkelgraden ist, wobei der zweite sich an den ersten Übergangszonenbereich (23) anschließende Übergangszonenbereich (24), ein sich verjüngender gerader Konus mit einem Kegelwinkel von 45 Winkelgraden ist und wobei der dritte sich an den zweiten Übergangszonenbereich (24) anschließende Übergangszonenbereich (25), ein sich verjüngender gerader Konus mit einem Kegelwinkel von 22,5 Winkelgraden ist.

4. Tordierinstrument gemäß Anspruch 1, **dadurch gekennzeichnet, dass** die an den Antriebsschaft (10) anschließende Halszylinderzone (31) eine Länge hat, die mindestens dem 6,6-fachen Durchmesser der Halszylinderzone (31) entspricht und dass der zwischen der Halszylinderzone (31) und dem Arbeitsteil (60) gelegene Halsabschnitt eine Länge hat, die mindestens das 5,5-fache des kleinsten Durchmessers dieses Halsabschnittes aufweist.

5. Tordierinstrument gemäß Anspruch 1, **dadurch gekennzeichnet, dass** die Sollbruchzone (40) im hinteren Bereich der an die Halsübergangszone (21) anschließenden Halszylinderzone (31) angeordnet ist.

6. Tordierinstrument gemäß Anspruch 1, **dadurch gekennzeichnet, dass** die Sollbruchzone (40) vier äquidistant auf dem Umfang der an die Halsübergangszone (21) anschließenden Halszylinderzone (31) verteilt angeordnete Abflachungen (41) aufweist.

7. Tordierinstrument gemäß Anspruch 6, **dadurch gekennzeichnet, dass** sich die nebeneinanderliegenden Abflachungen (41) der Sollbruchzone (40) gegenseitig nicht kontaktieren.

8. Tordierinstrument gemäß Anspruch 1, **dadurch gekennzeichnet, dass** der Kugelkopf (61) ein Außensechsrundkugelkopf ist.

## Claims

1. Twisting instrument for a screw-in component (90) with a tool recess (92), which comprises a drive shaft (10), a neck (20) and a working part (60),
- wherein the working part (60) - as a spherical head (61) having six teeth (63) - has a spherical enveloping surface (62) which is contacted at least both by the free end (76) and by partial regions of the tooth-own tooth heads (64) of the working part (60),
- wherein there are six tooth gaps (66) between the teeth (63),
- wherein the neck (20) or the drive shaft (10) has a predetermined breaking zone (40),
- wherein the neck (20) consists of a neck transition zone (21), a neck main cylinder zone (31), a neck cone zone (35) and a neck secondary cylinder zone (36), and
- wherein the spherical head (61) has a rear grip (67) at the transition of the spherical head (61) to the neck subcylinder zone (36), the radius contour of which merges tangentially into the neck subcylinder zone (36).

2. Twisting instrument according to claim 1, **characterised in that** the drive shaft (10) is of type 1 according to DIN EN ISO 1797, which is provided at its free end with a drive flattening (15) and a groove (17).

3. Twisting instrument according to claim 1, **characterised in that** the neck transition zone (21) comprises a first (23), second (24) and third transition zone region (25), wherein the first transition zone region (23) adjoining the drive shaft (10) is a tapering straight cone with a cone angle of 90 degrees, wherein the second transition zone region (24) adjoining the first transition zone region (23) is a tapering straight cone with a cone angle of 45 degrees of angle, and wherein the third transition zone region (25) adjoining the second transition zone region (24) is a tapering straight cone with a cone angle of 22.5 degrees of angle.

4. Twisting instrument according to claim 1, **characterised in that** the neck cylinder zone (31) adjoining the drive shaft (10) has a length which corresponds to at least 6.6 times the diameter of the neck cylinder zone (31) and **in that** the neck section located between the neck cylinder zone (31) and the working part (60) has a length which is at least 5.5 times the smallest diameter of this neck section.

5. Twisting instrument according to claim 1, **characterised in that** the predetermined breaking zone (40) is arranged in the rear region of the neck cylinder zone (31) adjoining the neck transition zone (21).

6. Twisting instrument according to claim 1, **characterised in that** the predetermined breaking zone (40) has four flattenings (41) arranged equidistantly on the circumference of the neck cylinder zone (31) adjoining the neck transition zone (21).

7. Twisting instrument according to claim 6, **characterised in that** the adjacent flattenings (41) of the predetermined breaking zone (40) do not contact each other.

8. Twisting instrument according to claim 1, **characterised in that** the spherical head (61) is an external hexagonal spherical head.

## Revendications

1. Instrument de torsion pour un composant à visser (90) avec un évidement d'outil (92), qui comprend une tige d'entraînement (10), un col (20) et une pièce de travail (60),
- la pièce de travail (60) - en tant que tête sphérique (61) présentant six dents (63) - ayant une surface d'enveloppe sphérique (62) qui est en contact au moins aussi bien avec l'extrémité libre (76) qu'avec des zones partielles des têtes de dents (64) propres aux dents de la pièce de travail (60),
- six espaces interdentaires (66) étant situés entre les dents (63),
- le col (20) ou la tige d'entraînement (10) présentant une zone de rupture (40),
- le col (20) étant constitué d'une zone de transition de col (21), d'une zone cylindrique principale de col (31), d'une zone conique de col (35) et d'une zone cylindrique secondaire de col (36), et
- la tête sphérique (61) possède, au niveau de la transition entre la tête sphérique (61) et la zone cylindrique secondaire de col (36), une poignée arrière (67) dont le contour du rayon se prolonge tangentiellement dans la zone cylindrique secondaire de col (36).

2. Instrument de torsion selon la revendication 1, **caractérisé en ce que** la tige d'entraînement (10) appartient au type 1 selon la norme DIN EN ISO 1797, qui est pourvue à son extrémité libre d'un méplat d'entraînement (15) et d'une rainure (17).

3. Instrument de torsion selon la revendication 1, **caractérisé en ce que** la zone de transition de col (21) comprend une première (23), une deuxième (24) et une troisième (25) région de zone de transition, la première région de zone de transition (23) se raccordant à la tige d'entraînement (10) étant un cône droit effilé avec un angle de cône de 90 degrés angulaires, la deuxième zone de transition (24) se raccordant à la première zone de transition (23) étant un cône droit effilé avec un angle de cône de 45 degrés d'angle et la troisième zone de transition (25) se raccordant à la deuxième zone de transition (24) étant un cône droit effilé avec un angle de cône de 22,5 degrés d'angle.

4. Instrument de torsion selon la revendication 1, **caractérisé en ce que** la zone cylindrique de col (31) se raccordant à la tige d'entraînement (10) a une longueur qui correspond au moins à 6,6 fois le diamètre de la zone cylindrique de col (31) et **en ce que** la section de col située entre la zone cylindrique de col (31) et la pièce de travail (60) a une longueur qui représente au moins 5,5 fois le plus petit diamètre de cette section de col.

5. Instrument de torsion selon la revendication 1, **caractérisé en ce que** la zone de rupture (40) est disposée dans la partie arrière de la zone cylindrique de col (31) qui se raccorde à la zone de transition de col (21).

6. Instrument de torsion selon la revendication 1, **caractérisé en ce que** la zone de rupture (40) présente quatre méplats (41) répartis de manière équidistante sur le pourtour de la zone cylindrique de col (31) se raccordant à la zone de transition de col (21).

7. Instrument de torsion selon la revendication 6, **caractérisé en ce que** les méplats (41) juxtaposés de la zone de rupture (40) ne sont pas en contact les uns avec les autres.

8. Instrument de torsion selon la revendication 1, **caractérisé en ce que** la tête sphérique (61) est une tête sphérique à six pans extérieurs.
